# EUROPEAN PATENT APPLICATION

(11) **EP 4 830 688 A1**
(43) Date of publication of application: **28.10.2026**
(21) Application number: 24907361.0
(22) Date of filing: 16.12.2024
(51) Int. Cl.: A61J 1/05

(54) **ANTIMICROBIAL COMPOSITION AND CONTAINER**

(30) Priority: 20.12.2023 JP 2023214677
(71) Applicant: Taisei Kako Co., Ltd., Kita-ku Osaka-shi Osaka 531-0072 (JP)
(72) Inventor: YANO, Yukihiro, Ibaraki-shi, Osaka 567-0054 (JP); JYO, Hanae, Ibaraki-shi, Osaka 567-0054 (JP); YAGI, Syoki, Ibaraki-shi, Osaka 567-0054 (JP); MIZUTANI, Moeka, Ibaraki-shi, Osaka 567-0054 (JP); NISHIO, Sayaka, Ibaraki-shi, Osaka 567-0054 (JP)
(74) Representative: Dehns
(86) International application number: PCT/JP2024/044389
(87) International publication number: WO 2025/134976

(57) **Abstract**

An embodiment of the present invention relates to an antibacterial composition containing a resin and an aluminum sulfate compound, or a pharmaceutical, quasi-drug, or cosmetic container formed from the antibacterial composition.

## Description

### Technical Field

An embodiment of the present invention relates to an antibacterial composition or a container.

### Background Art

In recent years, processed products formed of resins having functionality have increased, and in particular, antibacterial processed products formed of resins having an antibacterial function have been widely used even in general households. As means for imparting an antibacterial function to a resin, for example, a method of forming a coating film containing an antibacterial agent on a surface of the resin, and a method of mixing an antibacterial agent into the resin are known.

As the antibacterial agent, organic antibacterial agents and inorganic antibacterial agents are known, and from the viewpoints of high antibacterial effect and high safety, inorganic antibacterial agents such as silver-based antibacterial agents and zinc-based antibacterial agents are used.

For example, Patent Literature 1 discloses an eye drop container formed of a resin containing silver-supported zirconium phosphate.

### Citation List

### Patent Literature

Patent Literature 1: WO 2019/107569 A

### Summary of Invention

However, when an inorganic antibacterial agent is mixed into a resin, since the antibacterial agent is an inorganic solid, dispersibility in the organic resin is poor, and there have been problems in that the antibacterial effect is not sufficiently exhibited, or for example, discoloration (including, for example, white turbidity) or powdering occurs in the obtained composition.

An embodiment of the present invention provides an antibacterial composition that is a composition containing a resin and an inorganic antibacterial agent and has an excellent antibacterial effect and suppressed appearance defects.

### Solution to Problem

Examples of the configurations of the present invention are as follows.
[1] An antibacterial composition containing a resin and an aluminum sulfate compound.
[2] The antibacterial composition according to [1], in which a content of the aluminum sulfate compound is 0.09 to 5 parts by mass based on 100 parts by mass of the resin.
[3] The antibacterial composition according to [1] or [2], in which the resin contains an ethylene-based resin or a propylene-based resin.
[4] A pharmaceutical, quasi-drug, or cosmetic container formed from the antibacterial composition according to any one of [1] to [3].

### Advantageous Effects of Invention

According to an embodiment of the present invention, it is possible to provide an antibacterial composition that is a composition containing a resin and an inorganic antibacterial agent and has an excellent antibacterial effect and suppressed appearance defects.

According to an embodiment of the present invention, it is possible to provide an antibacterial composition capable of forming a container that satisfies requirements for an extractables test (pH) and residue on ignition specified in Notification Yakuhatsu No. 336.

### Brief Description of Drawings

Fig. 1 is an external front view showing an example of an eye drop container in which an inner plug is fitted into a container body.
Fig. 2 is an explanatory cross-sectional view showing an example of an eye drop container in which an inner plug is fitted into a container body.
Fig. 3 is an explanatory cross-sectional view showing an example of an eye drop container having a container body, an eye drop nozzle, and a cap.

### Description of Embodiments

### <<Antibacterial composition>>

An antibacterial composition according to an embodiment of the present invention (hereinafter, also referred to as "the present composition") contains a resin and an aluminum sulfate compound.

An antibacterial activity value of the present composition is preferably 2.0 or more.

The present composition having an antibacterial activity value within the above range can be said to have an excellent antibacterial effect.

Specifically, the antibacterial activity value is measured by the method described in the following Examples.

### <Resin>

The resin is not particularly limited, and various thermoplastic resins or thermosetting resins according to the intended use of the present composition can be used.

The resin used in the present composition may be one kind or two or more kinds.

Examples of the thermoplastic resin include an α-olefin-based resin (for example, an ethylene-based resin, a propylene-based resin), a cyclic olefin-based resin (for example, COP, COC), a vinylcyclohexane (block) copolymer, a diene-based resin (for example, polyisoprene, polybutadiene), a chlorine-containing resin (for example, chlorinated polyethylene, polyvinyl chloride), a styrene-based resin (including, for example, an acrylonitrile-styrene resin [AS resin], an acrylonitrile-butadiene-styrene resin [ABS resin], a methyl methacrylate-butadiene-styrene resin [MBS resin], a methyl methacrylate-acrylonitrile-butadiene-styrene resin [MABS resin], and an acrylonitrile-acrylic rubber-styrene resin [AAS resin]), a (meth)acrylic resin, an ester-based resin (for example, polyethylene terephthalate, polybutylene terephthalate, polyethylene naphthalate), polycarbonate, polyphenylene ether, modified polyphenylene ether, an amide-based resin (for example, an aliphatic polyamide, an aromatic polyamide), polyphenylene sulfide, polyimide, polyether ether ketone, polysulfone, polyarylate, polyether ketone, polyether nitrile, polythioether sulfone, polyether sulfone, polybenzimidazole, polyamide-imide, polyetherimide, polyacetal, a liquid crystal polymer, and a thermoplastic polyurethane.

Examples of the thermosetting resin include a polyurethane, a phenol resin, a melamine resin, a urea resin, an unsaturated polyester resin, a diallyl phthalate resin, a silicone resin, a fluorine resin, and an epoxy resin.

The resin may be a resin using only a biomass-derived raw material as a raw material (for example, olefin), a resin using only a fossil fuel-derived raw material, or a resin using both a biomass-derived raw material and a fossil fuel-derived raw material.

The resin is preferably a resin using a biomass-derived raw material from the viewpoint of reducing environmental load (mainly reducing greenhouse gases).

Among these, the resin is preferably a thermoplastic resin, more preferably an α-olefin-based resin, a cyclic olefin-based resin, or a vinylcyclohexane (block) copolymer, and still more preferably an α-olefin-based resin, from the viewpoint that a molded article having a desired shape (particularly a container) can be easily formed by a molding method such as injection molding.

### · α-Olefin-based resin

Preferred examples of the α-olefin-based resin include an ethylene-based resin and a propylene-based resin.

The ethylene-based resin is a resin containing a structural unit derived from ethylene as a main component, and specific examples thereof include a homopolymer of ethylene and a copolymer of ethylene and one or more other monomers other than ethylene (ethylene copolymer).

Specific examples of the ethylene-based resin include high-density polyethylene (HDPE), medium-density polyethylene (MDPE), low-density polyethylene (LDPE), and linear low-density polyethylene (LLDPE).

Examples of the other monomer include propylene and an α-olefin having 4 to 20 carbon atoms. Examples of the α-olefin having 4 to 20 carbon atoms include, for example, 1-butene, 1-pentene, 3-methyl-1-butene, 1-hexene, 4-methyl-1-pentene, 3-methyl-1-pentene, 1-octene, 1-decene, 1-dodecene, 1-tetradecene, 1-hexadecene, 1-octadecene, and 1-eicosene.

The other monomer is preferably propylene, 1-butene, 1-hexene, or 4-methyl-1-pentene, and more preferably propylene.

One kind or two or more kinds of the other monomer can be used.

The polypropylene-based resin is a resin containing a structural unit derived from propylene as a main component, and specific examples thereof include a homopolymer of propylene and a copolymer of propylene and one or more comonomers other than propylene (propylene copolymer).

The stereoregularity of the polypropylene-based resin is not particularly limited, and the propylene copolymer may be a random copolymer or may be a block copolymer, but is preferably a random copolymer.

Examples of the comonomer include ethylene and an α-olefin having 4 to 20 carbon atoms.

Examples of the α-olefin having 4 to 20 carbon atoms include, for example, α-olefins similar to the α-olefins having 4 to 20 carbon atoms mentioned in the section of the ethylene-based resin.

As the comonomer, ethylene, 1-butene, 1-hexene, and 4-methyl-1-pentene are preferable, and ethylene is more preferable.

One kind or two or more kinds of the comonomer can be used.

The content of the structural unit derived from the comonomer in the propylene copolymer is preferably 4.0 mass% or less, more preferably 3.8 mass% or less, and still more preferably 3.5 mass% or less, based on 100 mass% of all structural units constituting the propylene copolymer, from the viewpoint that, for example, the amount of impurities eluted from the present composition and a molded article obtained from the composition can be reduced, and the lower limit thereof is not particularly limited, but is, for example, 1.0 mass%.

### · Cyclic olefin-based resin

Examples of the cyclic olefin-based resin include COP and COC.

Examples of COP include a resin obtained by (co)polymerizing one kind or two or more kinds of cyclic olefins, or a hydrogenated product of the resin.

Examples of COC include a resin obtained by copolymerizing one kind or two or more kinds of cyclic olefins and one kind or two or more kinds of monomers copolymerizable with the cyclic olefins, or a hydrogenated product of the resin.

In addition, the cyclic olefin-based resin may be, for example, a graft-modified product obtained by modifying the resin or a hydrogenated product thereof with an unsaturated carboxylic acid, a derivative thereof, or other modifiers.

The cyclic olefin is not particularly limited, and examples thereof include, for example, compounds described in JP 2005-263287 A, for example, norbornene-based compounds such as norbornene, 6-methylnorbornene, 6-ethylnorbornene, 5-propylnorbornene, 6-n-butylnorbornene, 1-methylnorbornene, 7-methylnorbornene, 5,6-dimethylnorbornene, 5-phenylnorbornene, and 5-benzylnorbornene; tetracyclododecene-based compounds such as 8-methyltetracyclo-3-dodecene, 8-ethyltetracyclo-3-dodecene, and 5,10-dimethyltetracyclo-3-dodecene; cycloalkene-based compounds such as cyclobutene, cyclopentene, cyclohexene, 3,4-dimethylcyclopentene, 3-methylcyclohexene, 2-(2-methylbutyl)-1-cyclohexene, cyclooctene, and 3a,5,6,7a-tetrahydro-4,7-methano-1H-indene.

Examples of the monomer copolymerizable with the cyclic olefin include, for example, α-olefins having 2 to 20 carbon atoms such as ethylene, propylene, 1-butene, 1-pentene, 1-hexene, 3-methyl-1-butene, 3-methyl-1-pentene, 3-ethyl-1-pentene, 4-methyl-1-pentene, 4-methyl-1-hexene, 4,4-dimethyl-1-hexene, 4,4-dimethyl-1-pentene, 4-ethyl-1-hexene, 3-ethyl-1-hexene, 1-octene, 1-decene, 1-dodecene, 1-tetradecene, 1-hexadecene, 1-octadecene, and 1-eicosene; and non-conjugated dienes such as 1,4-hexadiene, 4-methyl-1,4-hexadiene, 5-methyl-1,4-hexadiene, and 1,7-octadiene.

### <Aluminum sulfate compound>

Examples of the aluminum sulfate compound include aluminum sulfate, a salt of aluminum sulfate, a hydrate of aluminum sulfate, and a hydrate of an aluminum sulfate salt.

The aluminum sulfate compound used in the present composition may be one kind or two or more kinds.

The aluminum sulfate means anhydrous aluminum sulfate.

Examples of the aluminum sulfate salt include, for example, sodium, potassium, and ammonium salts.

Examples of the hydrate include an octahydrate, a dodecahydrate, a 14- to 18-hydrate, and a 27-hydrate.

The content of the aluminum sulfate compound in the present composition is preferably 0.09 to 5 parts by mass, more preferably 0.09 to 3 parts by mass, and still more preferably 0.09 to 1 part by mass, based on 100 parts by mass of the resin, from the viewpoints that a composition and a molded article that have an excellent antibacterial effect and in which discoloration including white turbidity, powdering, and foaming, for example, are suppressed can be easily obtained.

The content of anhydrous aluminum sulfate and an anhydrous aluminum sulfate salt in the present composition is preferably 0.09 to 0.40 parts by mass, and more preferably 0.09 to 0.35 parts by mass, based on 100 parts by mass of the resin, from the viewpoints that the antibacterial effect of the aluminum sulfate compound, which is an antibacterial agent, is unlikely to decrease, and that a composition and a molded article having a residue on ignition value of 0.10% or less can be easily obtained, the residue on ignition value being tested in accordance with 1.1 Residue on Ignition of 1 Combustion Test of the Japanese Pharmacopoeia General Tests "Test Methods for Plastic Containers" specified in Notification Yakuhatsu No. 336.

The content of anhydrous aluminum sulfate and an anhydrous aluminum sulfate salt in the present composition is a value obtained by, when commercially available anhydrous aluminum sulfate or an anhydrous aluminum sulfate salt is used as the aluminum sulfate compound that is a raw material for preparing the present composition, considering the purity of the raw material (commercial product) in the blending amount of the raw material (commercial product), and is a value obtained by, when a commercially available aluminum sulfate hydrate or aluminum sulfate salt hydrate is used as the aluminum sulfate compound that is a raw material for preparing the present composition, considering the purity of the raw material (commercial product) and the amount of water in the hydrate in the blending amount of the raw material (commercial product).

### <Other components>

The present composition may contain, in addition to the resin and the aluminum sulfate compound, other components that have been used in conventional resin compositions, such as a pH adjuster, a stabilizer (for example, an antioxidant, a heat stabilizer, a light stabilizer, and a weather stabilizer), a colorant, a dispersant, a filler (for example, talc, calcium carbonate, silica, alumina, carbon black, zinc oxide, zeolite, hydrotalcite, glass fiber, paper powder, and wood powder), an antistatic agent, a lubricant, and a nucleating agent, as necessary for a desired use, within a range that does not impair the effects of the present invention.

The present composition may contain each of these other components alone or two or more kinds thereof.

### · pH adjuster

When the present composition is used for, for example, an eye drop container, the eye drop container may be required to satisfy the standards specified in Notification Yakuhatsu No. 336. Among them, the pH difference measured in accordance with 2 Extractables substances (II) pH of the Japanese Pharmacopoeia General Tests "Test Methods for Plastic Containers" may be required to be 1.5 or less, and from the viewpoints that a composition and a molded article having such a pH difference of 1.5 or less can be easily obtained, the present composition preferably contains a pH adjuster.

Examples of the pH adjuster include amine compounds (for example, alkanolamines such as dimethylethanolamine, diethanolamine, triethanolamine, and N-methyldiethanolamine; primary amines; secondary amines; tertiary amines; and quaternary ammonium salts); aqueous ammonia; alkali metal hydroxides such as lithium hydroxide, sodium hydroxide, and potassium hydroxide; and alkali metal carbonates or bicarbonates such as lithium carbonate, sodium carbonate, potassium carbonate, and sodium bicarbonate.

Among these, an amine compound is preferable, and an amine compound having a hindered amine structure is more preferable, from the viewpoints that the antibacterial effect of the aluminum sulfate compound, which is an antibacterial agent, is unlikely to decrease, the pH difference can be 1.5 or less, and a composition and a molded article having a residue on ignition value of 0.10% or less can be easily obtained, the residue on ignition value being tested in accordance with 1.1 Residue on Ignition of 1 Combustion Test of the Japanese Pharmacopoeia General Tests "Test Methods for Plastic Containers" specified in Notification Yakuhatsu No. 336.

The amine compound having a hindered amine structure is preferably a compound having two hydrocarbon groups each at the 2-position and the 6-position on a piperidine ring (four in total), more preferably a 2,2,6,6-tetraalkylpiperidine derivative, still more preferably a 2,2,6,6-tetramethylpiperidine derivative, and particularly preferably a 1-alkyl-2,2,6,6-tetramethylpiperidine derivative or a 1-hydro-2,2,6,6-tetramethylpiperidine derivative. Specific examples include a dimethyl succinate-1-(2-hydroxyethyl)-4-hydroxy-2,2,6,6-tetramethylpiperidine polycondensate, poly[{6-(1,1,3,3-tetramethylbutyl)amino-1,3,5-triazine-2,4-diyl}{(2,2,6,6-tetramethyl-4-piperidyl)imino}hexamethylene{(2,2,6,6-tetramethyl-4-piperidyl)imino}], an N,N'-bis(3-aminopropyl)ethylenediamine·2,4-bis[N-butyl-N-(1,2,2,6,6-pentamethyl-4-piperidyl)amino]-6-chloro-1,3,5-triazine condensate, and bis(2,2,6,6-tetramethyl-4-piperidyl) sebacate.

The number average molecular weight of the amine compound having a hindered amine structure is preferably 400 or more, and more preferably 400 to 5,000.

The melting point of the amine compound having a hindered amine structure is preferably 50 to 200°C, and more preferably 70 to 150°C.

When a pH adjuster is used in the present composition, the amount of the pH adjuster used is preferably 10 to 55 parts by mass, more preferably 12 to 53 parts by mass, and still more preferably 15 to 50 parts by mass, based on 1 part by mass of the aluminum sulfate compound, from the viewpoints that the antibacterial effect of the aluminum sulfate compound is unlikely to decrease, the pH difference can be 1.5 or less, and a composition and a molded article having a residue on ignition value of 0.10% or less can be easily obtained.

### <Method for preparing the present composition>

A method for preparing the present composition is not particularly limited, but a method of melt-kneading the respective components using a conventionally known apparatus, specifically, for example, a single-screw extruder, a twinscrew extruder, a kneader, a mixer, and a two-roll mill, is preferable.

For example, the order of adding the respective components to the apparatus at this time is not particularly limited.

### <<Molded article>>

The present composition is usually molded into a shape according to a desired use and used.

The shape of the molded article is not particularly limited, and examples thereof include a film (including a plate-like body such as a sheet, and may be stretched or unstretched), a fiber, a tube, and a container. According to an embodiment of the present invention, a molded article having an excellent antibacterial effect and suppressed appearance defects such as discoloration including white turbidity and powdering can be easily obtained, and therefore, among these, a container is preferable, a pharmaceutical, quasi-drug, or cosmetic container (a container for a pharmaceutical, quasi-drug, or cosmetic) is more preferable, and an eye drop container is still more preferable, from the viewpoint that, for example, these effects are further exhibited.

According to an embodiment of the present invention, a transparent molded article, particularly a transparent container, can be easily obtained. The container is preferable, for example, from the viewpoint of excellent visibility of contents thereof.

A colored molded article may be required depending on a desired use and from the viewpoint of design properties, but in this case, a conventionally known colorant may be used.

### <Configuration and other matters of eye drop container>

The eye drop container is not particularly limited, and examples thereof include a container having a container body, a cap, and an inner plug or an eye drop nozzle.

The container body is not particularly limited as long as it is a container body having a bottom portion, a hollow cylindrical body portion continuous with a peripheral edge of the bottom portion, a shoulder portion continuous with the body portion, and an opening cylindrical portion continuous with the shoulder portion and capable of containing an eye drop solution, and can have a conventionally known configuration.

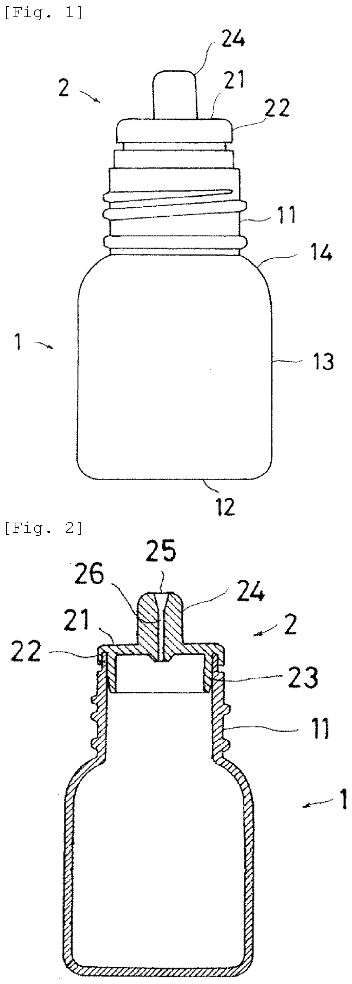

Specific examples of such a container body include the container bodies shown in Figs. 1 to 3.

Fig. 1 is an external front view showing an example of an eye drop container in which an inner plug is fitted, which is an embodiment of the container body, and Fig. 2 is an explanatory cross-sectional view of the container body.

A container body 1 in Fig. 1 includes a bottom portion 12, a hollow cylindrical body portion 13 continuous with a peripheral edge of the bottom portion 12, a shoulder portion 14 continuous with the body portion 13, and an opening cylindrical portion 11 having a threaded portion formed on an outer peripheral surface.

An inner plug 2 for liquid injection is fitted into the opening cylindrical portion 11 in Fig. 1.

The inner plug 2 generally includes a circular plate portion 21, an outer cylindrical portion 22 integral with the circular plate portion 21 and hanging down from a peripheral edge of the circular plate portion 21, an inner cylindrical portion 23 hanging down from a lower surface of the circular plate portion 21, and a columnar liquid injection portion 24 protruding from an upper surface of the circular plate portion 21, and the opening cylindrical portion 11 of the container body 1 is fitted into a gap between the outer cylindrical portion 22 and the inner cylindrical portion 23. A liquid injection port 25 is formed at a distal end of the liquid injection portion 24, and a liquid passage portion 26 through which an eye drop solution pushed out from the container body 1 passes is formed through from the liquid injection port 25 to the lower surface of the circular plate portion 21.

The container body 1 in Fig. 1 is usually used as an eye drop container by screwing a cap (not shown) onto the opening cylindrical portion 11 of the container body 1. The cap may be formed with a protruding portion that closes the liquid injection port 25 in a state in which the cap is attached.

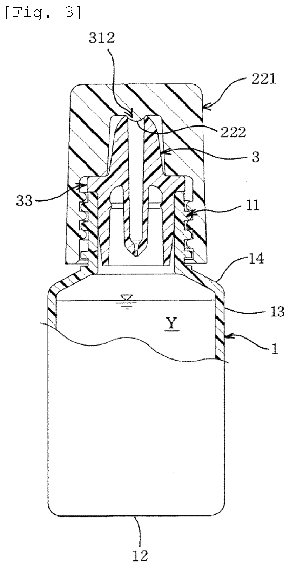

Fig. 3 is an explanatory cross-sectional view of an eye drop container having a container body 1, an eye drop nozzle 3, and a cap 221.

The eye drop container in Fig. 3 is composed of the container body 1 loaded with a predetermined amount (for example, 5 mL) of an eye drop solution (drug solution) Y, the eye drop nozzle 3 assembled to the opening cylindrical portion 11 of the container body 1, and the cap 221 that is screwed onto the opening cylindrical portion 11 and protects the eye drop nozzle (dropping nozzle) 3.

The container body 1 in Fig. 3 is composed of a bottom portion 12, a hollow cylindrical (bottle-shaped) body portion 13 continuous with a peripheral edge of the bottom portion 12, a shoulder portion 14 continuous with the body portion 13, and an opening cylindrical portion 11 having a threaded portion formed on an outer peripheral surface continuous with the shoulder portion.

The cap 221 has, on an inner peripheral surface, a threaded portion configured to allow the cap 221 to be detachably attached to a threaded portion on the outer peripheral surface of the opening cylindrical portion 11 by screwing, and a convex portion 222 capable of fitting into and sealing a distal end opening of a liquid injection port 312 of the eye drop nozzle 3 is formed on an upper inner surface. In a closed state in which the cap 221 is screwed onto the opening cylindrical portion 11 (the state shown in Fig. 3), the cap 221 closes the liquid injection port 312 with the convex portion 222 and can seal the inside of the container body 1 by coming into close contact with one or more of the upper surface of a flange portion 33 and the threaded portion.

Although Figs. 1 and 2 show a configuration in which the inner plug 2 and the container body 1 are separate bodies, and Fig. 3 shows a configuration in which the eye drop nozzle 3 and the container body 1 are separate bodies, the configuration is not limited thereto, and both may be integrally molded. In this case, filling of the eye drop solution Y contained in the container body 1 and molding of the container body 1 may be performed simultaneously.

The size of the container body is not particularly limited, and may be dimensionally designed so as to have a volume that can be filled with 1 to 20 mL of an eye drop solution. For example, the height of the container body is 30 to 50 mm, and the height of the body portion is, for example, 20 to 40 mm.

The average thickness of the body portion is, for example, 0.4 to 1.0 mm.

The shape of the bottom portion is not particularly limited, and examples thereof include a circular shape (including, for example, an ellipse and an oval shape) and a polygonal shape (including, for example, a rectangle, a rhombus, a pentagon, and a hexagon).

The diameter of the liquid injection port may be appropriately set according to the properties of the eye drop solution and is not particularly limited, but is usually dimensionally designed within a range of 1 to 4 mm.

The materials constituting the container body 1, the inner plug 2, the eye drop nozzle 3, and the cap are not particularly limited, and the present composition may be used in at least one of these, but the present composition is preferably used in all of these. Among these, as a material of a member in which the present composition is not used, a material that has been used for conventionally known members can be used, and examples thereof include injection-moldable thermoplastic resins such as polyethylene, polypropylene, and polyethylene terephthalate.

The method for producing, for example, the container body, the inner plug, the eye drop nozzle, and the cap is not particularly limited, and these members may be produced by a conventionally known production method. However, from the viewpoint that, for example, a container body and other members having a desired shape can be easily formed, these members are preferably produced by injection molding, direct blow molding, particularly injection (blow) molding.

### Examples

Hereinafter, an embodiment of the present invention will be described more specifically with reference to Examples, but the present invention is not limited thereto.

### [Example 1]

To an injection molding machine, 100 parts by mass of PP-1 (Prime Polypro J315FB, manufactured by Prime Polymer Co., Ltd., random polypropylene) was added, and 1.000 part by mass of an aluminum sulfate compound-1 was also added, and the mixture was melt-kneaded at 200°C to prepare an antibacterial composition.

The prepared antibacterial composition was injection-molded using an injection molding machine under conditions of a cylinder temperature of 200°C to prepare a container (inner plug 2) having the shape shown in Fig. 2.

### [Examples 2 to 5 and Comparative Examples 1 to 3]

Antibacterial compositions and containers (inner plugs) were prepared in the same manner as in Example 1, except that the type and amount of the antibacterial agent used were changed as shown in Table 1.

### [Examples 21 to 28]

Antibacterial compositions and containers (inner plugs) were prepared in the same manner as in Example 1, except that the types of the resin and the antibacterial agent were changed as shown in Table 2.

Details of the resins used in each test were as follows.
· "PP-1": Prime Polypro J315FB, manufactured by Prime Polymer Co., Ltd.
· "PP-2": NOVATEC PP MG2TA, manufactured by Japan Polypropylene Corporation
· "PP-3": SUMITOMO NOBLEN W531D, manufactured by Sumitomo Chemical Co., Ltd.
· "LDPE": SUMIKATHENE G801, manufactured by Sumitomo Chemical Co., Ltd.
· "HDPE-1": HI-ZEX HZ3000B, manufactured by Prime Polymer Co., Ltd.
· "HDPE-2": SHC7260, manufactured by Braskem S.A.
· "SB": ZELAS MC932, manufactured by Mitsubishi Chemical Corporation
· "COC": 6013S-04, manufactured by Polyplastics Co., Ltd.

Details of the antibacterial agents used in each test were as follows.
· "Aluminum sulfate compound-1": manufactured by KISHIDA CHEMICAL CO., LTD., aluminum sulfate (dehydrated), for chemical use
· "Aluminum sulfate compound-2": manufactured by Goto Sangyo Co., Ltd., burnt alum
· "Aluminum sulfate compound-3": manufactured by FUJIFILM Wako Pure Chemical Corporation, aluminum sulfate 14-18 hydrate
· "BACTEKILLER": BM-102TG (Ag-Zn-based antibacterial agent), manufactured by Fuji Chemical Industries Ltd.
· "Zeoal": Zeaol AgA50 (silver ion-supported zeolite-based antibacterial agent), manufactured by Nakamura Choukou Co., Ltd.

### <Antibacterial test (antibacterial activity value)>

A shake method antibacterial test was conducted in accordance with the shake method of (2) Test Method II (2012 edition) in "2. Antibacterial Power Evaluation Test Methods for Antibacterial Products" of the Society of International sustaining growth for Antimicrobial Articles.

Specifically, the antibacterial test was conducted as follows.

A nutrient broth (NB) medium (manufactured by Nissui Pharmaceutical Co., Ltd.) was diluted 500-fold with purified water and adjusted to a pH of 7.0±0.2 to prepare a "1/500 NB medium".

In addition, two pellets of ATCC 8739 (manufactured by Microbiologics, EZ-PECTM), which is an E. coli (Escherichia coli) strain, were transferred to a vial containing 2 mL of a hydration solution and placed in a constant-temperature bath at 34 to 38°C for 30 minutes to prepare a suspension.

Further, from the containers (inner plugs) prepared in Examples 1 to 5, Examples 21 to 28, and Comparative Examples 1 to 3, test pieces (antibacterial processed test pieces) were cut out so that a total surface area was 32±5 cm².

In Example 1, except that aluminum sulfate was not used, a container (inner plug) was prepared in the same manner as in Example 1, and test pieces (untreated test pieces) were cut out from the container (inner plug) so that a total surface area was 32±5 cm².

The suspension prepared above was added to the prepared 1/500 NB medium so that the number of bacteria was 10⁴ to 10⁵, thereby preparing a bacterial solution for inoculation.

10 mL of the bacterial solution for inoculation and the test piece were placed in a sterilized cup, and shake-cultured in a constant-temperature bath at 35±1°C and 150±10 rpm (horizontal direction) for 24 hours to prepare a cultured bacterial solution. Using 0.85 mass% physiological saline, the prepared cultured bacterial solution was diluted 10-fold, 10²-fold, 10³-fold, 10⁴-fold, 10⁵-fold, 10⁶-fold, and 10⁷-fold.

1 mL of each dilution diluted at each dilution ratio was placed in a sterilized petri dish, 15 mL of warmed standard agar medium (manufactured by Nissui Pharmaceutical Co., Ltd.) was added thereto, the mixture was cultured in a constant-temperature bath at 35°C for 24 hours, and the number of viable bacteria was calculated from the number of colonies on the medium.

In addition, the number of viable bacteria was calculated from the number of colonies on the medium in the same manner as described above, except that ATCC 6538TM (Microbiologics, EZ-PECTM), which is an S. aureus (Staphylococcus aureus) strain, was used instead of ATCC 8739.

The "antibacterial activity value" for each of E. coli and S. aureus was calculated according to the following formula, and a value obtained by truncating the second decimal place was shown in Table 1 or Table 2. When the antibacterial activity value is 2.0 or more, it is determined that "there is an antibacterial effect".

Antibacterial activity value=(Ut-U0)-(At-U0)=Ut-At
U0: average value of logarithmic values of the number of viable bacteria immediately after inoculation when the untreated test piece was used
Ut: average value of logarithmic values of the number of viable bacteria after 24 hours when the untreated test piece was used
At: average value of logarithmic values of the number of viable bacteria after 24 hours when the antibacterial processed test piece was used

### <Appearance evaluation>

The appearance of the containers (inner plugs) prepared in Examples 1 to 5, Examples 21 to 28, and Comparative Examples 1 to 3 was visually evaluated according to the following evaluation criteria. The results are shown in Table 1 or Table 2.
OO: the obtained container was transparent (no discoloration including white turbidity occurred), and no powdering occurred
O: the obtained container was transparent but was light brown, and no powdering occurred
Δ: the obtained container was slightly white turbid, but no powdering occurred
X: discoloration such as white turbidity or powdering occurred in the obtained container

**[Table 1]**

| | Resin | Antibacterial agent | | Antibacterial activity value | | Appearance |
|---|---|---|---|---|---|---|
| | | Type | Amount used (parts by mass) | E. coli | S. aureus | |
| Example 1 | PP-1 | Aluminum sulfate compound-1 | 1.000 | 7.6 or more | | OO |
| Example 2 | PP-1 | Aluminum sulfate compound-2 | 1.000 | 5.1 | | o |
| Example 3 | PP-1 | Aluminum sulfate compound-1 | 0.230 | 7.5 or more | 6.5 | OO |
| Example 4 | PP-1 | Aluminum sulfate compound-1 | 0.138 | 7.6 or more | | OO |
| Example 5 | PP-1 | Aluminum sulfate compound-1 | 0.115 | 7.8 or more | 6.2 | OO |
| Comparative Example 1 | PP-1 | BACTEKILLER | 0.230 | 0.3 | | o |
| Comparative Example 2 | PP-1 | Zeaol | 0.230 | 0 | | Δ |
| Comparative Example 3 | PP-1 | Calcium hydroxide | 0.280 | Less than 2.0 | | X |

**[Table 2]**

| | Resin | Antibacterial agent | | Antibacterial activity value | Appearance |
|---|---|---|---|---|---|
| | | Type | Amount used (parts by mass) | E. coli | |
| Example 21 | PP-1 | Aluminum sulfate compound-3 | 0.310 | 7.5 | OO |
| Example 22 | PP-2 | Aluminum sulfate compound-3 | 0.310 | 5.2 | OO |
| Example 23 | PP-3 | Aluminum sulfate compound-3 | 0.310 | 6 | OO |
| Example 24 | LDPE | Aluminum sulfate compound-3 | 0.310 | 3 | OO |
| Example 25 | HDPE-1 | Aluminum sulfate compound-3 | 0.310 | 4 | OO |
| Example 26 | HDPE-2 | Aluminum sulfate compound-3 | 0.310 | 4.9 | OO |
| Example 27 | SB | Aluminum sulfate compound-3 | 0.310 | 4.3 | OO |
| Example 28 | COC | Aluminum sulfate compound-3 | 0.310 | 4.4 | OO |

### [Example 6]

To an injection molding machine, 100 parts by mass of polypropylene (Prime Polypro J315FB, manufactured by Prime Polymer Co., Ltd., random polypropylene), 0.23 parts by mass of the aluminum sulfate compound-1, and 2 parts by mass of HALS (Tinuvin 770DF, manufactured by BASF Japan Ltd.) were added, and the mixture was melt-kneaded at 200°C to prepare an antibacterial composition.

The prepared antibacterial composition was injection-molded using an injection molding machine under conditions of a cylinder temperature of 200°C to prepare a container (inner plug 2) having the shape shown in Fig. 2.

### [Examples 7 and 8]

Containers (inner plugs 2) were prepared in the same manner as in Example 6, except that the amount of HALS used was changed as shown in Table 3.

### [Example 9]

To an injection molding machine, 100 parts by mass of polypropylene (Prime Polypro J315FB, manufactured by Prime Polymer Co., Ltd., random polypropylene), 0.18 parts by mass of the aluminum sulfate compound-1, and 0.27 parts by mass of sodium bicarbonate (manufactured by KENEI Pharmaceutical Co., Ltd.) were added, and the mixture was melt-kneaded at 200°C to prepare an antibacterial composition.

The prepared antibacterial composition was injection-molded using an injection molding machine under conditions of a cylinder temperature of 200°C to prepare a container (inner plug 2) having the shape shown in Fig. 2.

### <Extractables Test (pH)>

An extractables test (pH) was performed in accordance with 2 Extractables substances (II) pH of the Japanese Pharmacopoeia General Tests "Test Methods for Plastic Containers".

Specifically, the test was performed as follows.

Test pieces were cut out from each of the containers (inner plugs) prepared in Examples 6 to 9 so that the total surface area of the front and back surfaces was 300 cm², and the test pieces were further cut into pieces having a length of 3 cm and a width of 0.3 cm. All the cut test pieces were placed in a 200 mL hard glass container. Thereafter, 100 mL of purified water was added to the hard glass container and heated at 70°C for 24 hours to obtain a test solution.

In addition, a blank test solution was obtained in the same manner as described above, except that no test piece was placed in the hard glass container.

20 mL each of the obtained test solution and blank test solution was taken, and 1.0 mL of a solution prepared by dissolving 1.0 g of potassium chloride in water to make 1,000 mL was added to each of the 20 mL of the test solution and the blank test solution. Thereafter, the pH of both solutions was measured, and the difference therebetween was calculated. The results are shown in Table 3.

In Notification Yakuhatsu No. 336, this pH difference is specified to be 1.5 or less.

### <Residue on ignition>

A test was performed in accordance with 1.1 Residue on Ignition of 1 Combustion Test of the Japanese Pharmacopoeia General Tests "Test Methods for Plastic Containers".

Specifically, the test was performed as follows.

A crucible was ignited in advance at 600±50°C for 30 minutes, and after cooling, the mass of the crucible was precisely measured.

Each of the containers (inner plugs) prepared in Examples 6 to 9 was finely pulverized, and 5 g of the pulverized product was placed in the crucible whose mass had been measured, and the mass of the pulverized product was precisely measured.

1 mL of sulfuric acid was added to the crucible containing the pulverized product whose mass had been measured, and the crucible was gradually heated to completely carbonize the sample in the crucible. Next, after cooling to room temperature, 1 mL of sulfuric acid was added again, the crucible was gradually heated until white smoke was no longer generated, and was further ignited at 600±50°C to ash the residue in the crucible. After cooling to room temperature, the mass of the obtained ash was precisely measured. The residue on ignition was calculated from the following formula. The results are shown in Table 3. 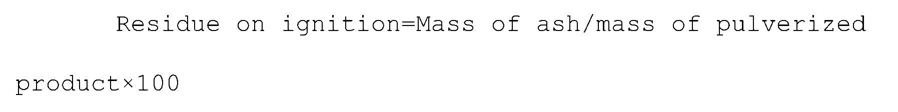 Residue on ignition=Mass of ash/mass of pulverized product×100

In Notification Yakuhatsu No. 336, this residue on ignition is specified to be 0.1% or less.

### <Appearance evaluation>

The appearance of the containers (inner plugs) prepared in Examples 6 to 9 was visually evaluated according to the same evaluation criteria as in the appearance evaluation of Example 1 and other examples. The results are shown in Table 3.

**[Table 3]**

| | Antibacterial agent | | Neutralizing agent | | pH difference | Residue on ignition (%) | Appearance |
|---|---|---|---|---|---|---|---|
| | Type | Amount used (parts by mass) | Type | Amount used (parts by mass) | | | |
| Example 6 | Aluminum sulfate compound-1 | 0.23 | HALS | 2 | 2.16 | 0.10 | OO |
| Example 7 | Aluminum sulfate compound-1 | 0.23 | HALS | 5 | 0.5 or less | 0.08 | OO |
| Example 8 | Aluminum sulfate compound-1 | 0.23 | HALS | 10 | 0.5 or less | - | o |
| Example 9 | Aluminum sulfate compound-1 | 0.18 | Sodium bicarbonate | 0.27 | 1.4 | 0.31 | OO |

### [Example 10]

To an injection molding machine, 100 parts by mass of polypropylene (Prime Polypro J315FB, manufactured by Prime Polymer Co., Ltd., random polypropylene) and 0.23 parts by mass of the aluminum sulfate compound-3 were added, and the mixture was melt-kneaded at 200°C to prepare an antibacterial composition.

In the prepared antibacterial composition, the amount of anhydrous aluminum sulfate excluding impurities, considering the purity and the amount of water in the hydrate, was 0.1247 parts by mass.

The prepared antibacterial composition was injection-molded using an injection molding machine under conditions of a cylinder temperature of 200°C to prepare a container (inner plug 2) having the shape shown in Fig. 2.

### [Examples 11 and 12]

In Example 10, a container (inner plug 2) was prepared in the same manner as in Example 10, except that the type and amount of the aluminum sulfate compound used were changed as shown in Table 4.

### <Residue on ignition>

The residue on ignition of the containers (inner plugs) prepared in Examples 10 to 12 was measured in the same manner as in Example 6 and other examples. The results are shown in Table 4.

**[Table 4]**

| | Antibacterial agent | | Amount of anhydrous aluminum sulfate (parts by mass) | Residue on ignition (%) |
|---|---|---|---|---|
| | Type | Amount used (parts by mass) | | |
| Example 10 | Aluminum sulfate compound-3 | 0.23 | 0.1247 | 0.057 |
| Example 11 | Aluminum sulfate compound-1 | 0.23 | 0.1609 | 0.080 |
| Example 12 | Aluminum sulfate compound-1 | 1.00 | 0.6996 | 0.385 |

### Reference Signs List

- 1: Container body
- 2: Inner plug
- 3: Eye drop nozzle
- 11: Opening cylindrical portion
- 12: Bottom portion
- 13: Body portion
- 14: Shoulder portion
- 21: Circular plate portion
- 22: Outer cylindrical portion
- 23: Inner cylindrical portion
- 24: Liquid injection portion
- 25: Liquid injection port
- 26: Liquid passage portion
- 33: Flange portion
- 221: Cap
- 222: Convex portion
- 312: Liquid injection port
- Y: Eye drop solution (drug solution)

## Claims

1. An antibacterial composition comprising a resin and an aluminum sulfate compound.

2. The antibacterial composition according to claim 1, wherein a content of the aluminum sulfate compound is 0.09 to 5 parts by mass based on 100 parts by mass of the resin.

3. The antibacterial composition according to claim 1, wherein the resin contains an ethylene-based resin or a propylene-based resin.

4. A pharmaceutical, quasi-drug, or cosmetic container formed from the antibacterial composition according to any one of claims 1 to 3.
